# NEUE EUROPÄISCHE PATENTSCHRIFT

(11) **EP 1 542 954 B2**
(45) Veröffentlichungstag und Bekanntmachung des Hinweises auf die Entscheidung über den Einspruch: **02.12.2015**
(45) Hinweis auf die Patenterteilung: 02.01.2008
(21) Anmeldenummer: 03725100.6
(22) Anmeldetag: 25.04.2003
(51) Int. Cl.: C07C 41/03, C07C 43/11, C11D 1/72, C11D 1/722

(54) **HERSTELLUNG VON ALKOXYLATEN AUF DER BASIS VON ETHYLENOXID UND 2-PROPYLHEPTANOL IN GEGENWART EINES INERTGASES**
METHOD FOR PRODUCING ALKOXYLATES FROM ETHYLENE OXIDE AND 2-PROPYLHEPTANOL IN THE PRESENCE OF AN INERT GAS
PRODUCTION D'ALCOXYLATS A PARTIR D'OXYDE D'ETHYLENE ET DE 2-PROPYLHEPTANOL EN PRESENCE D'UN GAZ INERTE

(30) Priorität: 18.09.2002 DE 10243362
(43) Veröffentlichungstag der Anmeldung: 22.06.2005
(73) Patentinhaber: BASF SE, 67056 Ludwigshafen (DE)
(72) Erfinder: WULFF, Christian, 68163 Mannheim (DE); STÖSSER, Michael, 67141 Neuhofen (DE); GROSCH, Georg, Heinrich, 2930 Brasschaat (BE); BALDENIUS, Kai-Uwe, 67063 Ludwigshafen (DE); BOHRES, Edward, 68161 Mannheim (DE)
(74) Vertreter: Isenbruck, Günter
(86) Internationale Anmeldenummer: PCT/EP2003/004331
(87) Internationale Veröffentlichungsnummer: WO 2004/033403

(56) Entgegenhaltungen:
- WO-A-00/74845
- WO-A-94/11330
- US-A- 2 508 036

## Beschreibung

Die vorliegende Erfindung betrifft Verfahren zur Herstellung mindestens eines Alkoxylats, umfassend das Inkontaktbringen von Ethylenoxid mit 2-Propylheptanol oder einem Isomerengemisch davon in Gegenwart mindestens einer Doppelmetallcyanid-Verbindung, wobei die Umsetzung bei einer Temperatur von 130°C bis 155°C erfolgt, und ein Inertgas zugegeben wird, wobei der Partialdruck des Inertgases während der Umsetzung 1,5 bis 20 bar beträgt.

Aus der Literatur ist bekannt, dass Doppelmetallcyanid-Verbindungen (DMC-Verbindungen) als Katalysatoren zur Umsetzung von Startermolekülen mit aktivem Wasserstoff und Alkylenoxiden, beispielsweise in einer Polymerisationsreaktion eingesetzt werden können. Die Ring-öffnende Polymerisationen von Alkylenoxiden wird beispielsweise in der EP-A 0 892 002, EP-A 0 862 977 und in der EP-A 0 755 716 beschrieben. DMC-Verbindungen weisen bei der Polymerisation von Alkylenoxiden eine hohe Aktivität als Katalysator auf.

Verfahren zur Alkoxylierung von aliphatischen Alkoholen sowie die erhaltenen Alkoxylate sind prinzipiell aus dem Stand der Technik bekannt. In der WO 01/04183 wird beispielsweise ein Verfahren zur Ethoxylierung von hydroxyfunktionellen Starterverbindungen beschrieben, das in Gegenwart einer Doppelinetallcyanid-Verbindung als Katalysator durchgeführt wird.

Alkoxylate von aliphatischen Alkoholen werden in großem Umfang als Tenside, Emulgatoren oder Schaumdämpfer eingesetzt. Die Benetzungs- und Emulgatoreigenschaften hängen dabei stark von der Art des Alkohols und der Art und Menge der Alkoxid-Addukte ab.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid in Gegenwart von Alkalihydroxiden als Katalysator umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt. WO 94/11331 betrifft die Verwendung derartiger Alkoxylate.

US 2,508,036 betrifft ebenfalls die Verwendung von 2-n-Propylheptanolethoxylaten, die 5 bis 15 mol Ethylenoxid enthalten, als Netzmittel in wässrigen Lösungen. Es ist beschrieben, dass die Produkte als Tenside in Waschmitteln eingesetzt werden können.

Die DE-A-102 18 754 sowie die DE-A-102 18 753 betreffen die Verwendung von C₁₀-Alkanolalkoxylat-Gemischen, insbesondere Alkanolethoxylat-Gemische, derartige C₁₀-Alkanolalkoxylat-Gemische und Verfahren zu ihrer Herstellung. Die DE-A-102 18 752 beschreibt ebenfalls Alkoxylat-Gemische und diese enthaltende Waschmittel wie auch Verfahren zur Herstellung der Alkoxylat-Gemische und die Verwendung des Waschmittels zum Waschen oder Reinigen von Textilien.

Bei der Alkoxylierung, insbesondere der Ethoxylierung, von Starterverbindungen in Gegenwart von Doppelmetallcyanid-Verbindungen treten insbesondere zwei Schwierigkeiten auf. Zum einen ist die Induktionsphase der Reaktion zum Teil sehr lang, was zu einer Verlängerung der Reaktionszeiten und zu erhöhten Kosten führt, zum anderen lässt die Aktivität des Katalysators während der Reaktion häufig langsam nach bis keine ausreichende Reaktionsgeschwindigkeit mehr vorhanden ist. Damit verläuft die Umsetzung nicht vollständig und die erhaltenen Produkte enthalten Verunreinigungen, beispielsweise Reste der Starterverbindung.

Eine Aufgabe der vorliegenden Reaktion bestand daher darin, ein Verfahren zur Alkoxylierung von Starterverbindungen mit verbesserten Reaktionsgeschwindigkeiten, verbessertem Umsatz, verbesserter Katalysator-Stabilität und verkürzter Induktionszeit bereitzustellen.

Erfindungsgemäß wird diese Aufgabe gelöst durch ein Verfahren zur Herstellung mindestens eines Alkoxylats, umfassend das Inkontaktbringen von Ethylenoxid mit 2-Propylheptanol oder einem Isomerengemisch davon in Gegenwart mindestens einer Doppelmetallcyanid-Verbindung der allgemeinen Formel I:

M¹ₐ[M²(CN)_{b}(A)_{c}]_{d}·fM¹_{g}Xₙ·h(H₂O)·eL·kP (I),

in der
- M¹ mindestens ein Metallion, ausgewählt aus der Gruppe bestehend aus Zn²⁺ Fe²⁺, Fe³, Co³⁺, Ni²⁺, Mn²⁺, Qo²⁺, Sn²⁺, Pb²⁺, Mo⁴⁺, Mo⁶⁺, Al³⁺, V⁴⁺, V⁵⁺, Sr^{2*}, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sek-undären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind,
wobei die Umsetzung bei einer Temperatur von 130°C bis 155°C erfolgt, und ein Inertgas zugegeben wird, wobei der Partialdruck des Inertgases während der Umsetzung 1,5 bis 20 bar beträgt.

Erfindungsgemäß erfolgt die Umsetzung von Ethylenoxid mit 2-Propylheptanol oder einem Isomerengemisch davon bei einer Temperatur von 130°C bis 155°C, bevorzugt von 140°C bis 155°C, besonders bevorzugt von 140°C bis 150°C.

Die gesamte Umsetzung erfolgt in dem genannten Temperaturbereich. Dies führt dazu, dass die Induktionsphase gegenüber der konventionellen Fahrweise bei niedrigeren Temperaturen verkürzt wird und die Standzeit des Katalysators verbessert wird. Bei höheren Temperaturen als dem genannten Temperaturbereich kann es insbesondere bei der Ethoxylierung zu einer Verschlechterung der Katalysator-Aktivität während der Reaktion kommen. Damit verlaufen die Umsetzungen mit höheren Ausbeuten und Produkte mit vermindertem Anteil an Restalkohol sowie an alkoxylierten Verbindungen mit niedrigem Alkoxylierungsgrad werden erhalten. Weiterhin erniedrigt sich der Restalkohol-Gehalt überraschenderweise auch bei gleichem Alkoxylierungsgrad der Produkte, wenn man die Reaktionstemperatur auf mindestens 130 °C, bevorzugt mindestens 140 °C erhöht.

Die Umsetzung kann dabei bei konstanter Temperatur oder aber mit einem bestimmten Temperaturprofil, beispielsweise einer während der Umsetzung ansteigenden Temperatur, gefahren werden, wobei im vorher definierten Temperaturbereich gearbeitet wird.

Prinzipiell ist die Temperatur während der Induktionsperiode frei wählbar. Sie kann jedoch auch während der Induktionsperiode im Bereich von 130°C bis 155°C liegen. Unter einer Induktionsperiode wird verstanden, dass die Alkoxylierungsreaktion nach dem Inkontaktbringen des Ethylenoxids mit 2-Propylheptanol oder einem Isomerengemisch davon und der Doppelmetallcyanidverbindung nicht sofort beginnt, sondern um eine gewisse Zeit verzögert ist. Diese Induktionsperiode äußerst sich beispielsweise dadurch, dass nach der Dosierung einer kleinen Menge Ethylenoxid ein gewisser Druck im Reaktor entsteht, der für eine gewisse Zeit konstant bleibt und am Ende der Induktionsperiode schnell abfällt. Nach dem Druckabfall ist die Reaktion angesprungen, und die weitere Dosierung des Ethylenoxids kann erfolgen.

In einer bevorzugten Ausführungsform wird in dem erfindungsgemäßen Verfahren zunächst der Reaktor mit einem Inertgas beaufschlagt, und anschließend wird das Ethylenoxid zugegeben. Diese Fahrweise hat den Vorteil, dass die Konzentration des Ethylenoxids in der Gasphase so niedrig gehalten werden kann, dass der Gasphasenzerfall des Ethylenoxids reduziert, insbesondere weitgehend vermieden werden kann.

Bei dem erfindungsgemäßen Verfahren kann beispielsweise zunächst das Reaktionsgefäß mit einer Suspension aus Alkohol und DMC-Katalysator befüllt werden. Anschließend kann der Katalysator durch Abtrennen von Wasser, z. B. durch Erhitzen und/oder Evakuieren des Reaktionsgefäßes, aktiviert werden.

Anschließend wird das Reaktionsgemisch vorteilhafterweise auf Reaktionstemperatur erwärmt, und ein Stickstoff-Vordruck wird eingestellt. Im weiteren Verlauf des Verfahrens wird beispielsweise eine Startmenge Ethylenoxid zudosiert. Nach dem Anspringen der Reaktion wird weiteres Ethylenoxid zudosiert, das Reaktionsgemisch wird gerührt, bis alles Ethylenoxid abreagiert ist. Das Reaktionsgemisch kann gegebenenfalls weiter aufgearbeitet werden.

Neben Ethylenoxid wird ein Inertgas zugegeben. Geeignete Inertgase sind im Rahmen der vorliegenden Erfindung beispielweise Stickstoff, CO₂ oder Edelgase wie Argon oder Gemische davon, bevorzugt Stickstoff.

Der Partialdruck des Inertgases beträgt während der Umsetzung 1.5 bis 20 bar, bevorzugt 1.5 bis 10 bar, insbesondere 1.5 bis 6 bar.

Der Druck des Ethylenoxids beträgt während der Umsetzung 0 bis 10 bar, bevorzugt 0 bis 6 bar, insbesondere 0 bis 3 bar.

Erfindungsgemäß ändert sich der Druck während der Reaktion. Beim Anspringen der Reaktion fällt der Druck anfänglich etwas ab. Dies ist jedoch abhängig von der Dosiergeschwindigkeit des Ethylenoxids. Wenn ein Inertgas vorhanden ist, wird dieses bei steigendem Füllstand des Reaktors komprimiert und mindestens sein Partialdruck erhöht sich.

Es ist im Rahmen einer Ausführungsform der vorliegenden Erfindung bevorzugt, dass die Summe aus Inertgas-Partialdruck und Ethylenoxid-Partialdruck während der Induktionsphase bei 0 bis 20 bar, beispielsweise bei 0 bis 10 bar, insbesondere bei 1,5 bis 6,0 bar, bevorzugt bei 1,5 bis 5,0 bar, besonders bevorzugt bei 1,5 bis 3,0 bar liegt.

In einer weiteren Ausführungsform betrifft die vorliegende Erfindung daher ein Verfahren, wobei die Umsetzung im oben definierten Temperaturbereich erfolgt und während der Induktionsphase die Summe aus Inertgas-Partialdruck und Ethylenoxid-Partialdruck bei 1,5 bar bis 6,0 bar liegt.

Im erfindungsgemäßen Verfahren wird Ethylenoxid eingesetzt.

Aus Sicherheitsgründen sollte eine Konzentration von >40%, bevorzugt >50% Ethylenoxid in der Gasphase im Reaktor vermieden werden, da es bei hohen Konzentrationen zum spontanen EO-Zerfall und damit einer Überhitzung oder Explosion des Reaktors kommen kann. Ethylenoxid wird daher im Rahmen der vorliegenden Erfindung mit einem Inertgas vermischt.

Das erfindungsgemäßen Verfahren zur Herstellung eines Alkoxylats wird in Gegenwart einer Doppelmetallcyanid-Verbindung der allgemeinen Formel I als Katalysator durchgeführt:

M¹ₐ[M²(CN)_{b}(A)_{c}]_{d}·fM¹_{g}Xₙ·h(H₂O)·eL·kP (I),

in der
- M¹ mindestens ein Metallion, ausgewählt aus der Gruppe bestehend aus Zn²⁺, Fe²⁺ Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Mg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺, ist,
- M² mindestens ein Metallion, ausgewählt aus der Gruppe bestehend aus Fe²⁺ Fe³⁺, Co²⁺, Co³⁺, Mn²⁺, Mn³⁺, V⁴⁺, V⁵⁺ Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

In einer bevorzugten Ausführungsform betrifft die vorliegende Erfindung daher ein Verfahren, bei dem die als Katalysator eingesetzte Doppelmetallcyanid-Verbindung kristallin ist.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP001/01893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration bezogen auf das Endmengengerüst ist typischerweise kleiner als 2000 ppm (d.h. mg Katalysator pro kg Produkt), bevorzugt kleiner als 1000 ppm, insbesondere kleiner als 500 ppm, besonders bevorzugt kleiner als 100 ppm, beispielsweise kleiner als 50 ppm oder kleiner als 35 ppm, insbesondere bevorzugt kleiner als 25 ppm.

In einer bevorzugten Ausführungsform betrifft die vorliegende Erfindung ein Verfahren, bei dem die Doppelmetallcyanid-Verbindung in einer Menge von 100 ppm oder weniger bezogen auf das Endmengengerüst eingesetzt wird.

In weiteren Ausführungsformen betrifft die vorliegende Erfindung ein Verfahren, wobei mindestens eine der folgenden Eigenschaften erfüllt ist:
1. (1) M¹ ist ausgewählt aus der Gruppe Zn²⁺, Fe²⁺, Fe³⁺, Co³⁺, Ni²⁺, Mn²⁺, Co²⁺;
2. (2) M² ist ausgewählt aus der Gruppe Fe²⁺, Fe³⁺, Co³⁺,
oder besonders bevorzugt ein Verfahren, wobei M¹ Zn²⁺ ist und M² Co³⁺.

Als Starter-Verbindung wird erfindungsgemäß 2-Propylheptanol oder ein Isomerengemisch davon eingesetzt.

Bei den als Starterverbindung eingesetzten Alkoholen kann es sich erfindungsgemäß auch um Gemische verschiedener Isomere handeln. Beispielsweise kann Propylheptanol ausgehend von Valeraldehyd durch Aldolkondensation und nachfolgende Hydrierung erhalten werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 und Römpp, Chemie Lexikon, 9. Auflage, Stichwort "Aldol-Addition" Seite 91. Die Hydrierung des Aldolkondensationsproduktes folgt allgemeinen Hydrierbedingungen.

Unter einem Isomerengemisch von 2-Propylheptanol wird im Rahmen der vorliegenden Anmeldung die Mischung von 2-Propylheptanol mit primären aliphatischen Alkoholen der gleichen Summenformel verstanden. Bevorzugt sind dies Verbindungen, die bei der Herstellung von 2-Propylheptanol als Nebenprodukte anfallen.

Des weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z.B. Marcel Guerbet, C.R. Acad Sci Paris 128, 511, 1002 (1899). Des weiteren ist auf Römpp, Chemie Lexikon, 9. Auflage, Georg Thieme Verlag Stuttgart, und die dort genannten Zitate sowie Tetrahedron, Vol. 23, Seiten 1723 bis 1733, hinzuweisen.

Vorzugsweise wird das Ethylenoxid im erfindungsgemäßen Verfahren in solchen Mengen eingesetzt, das der erhaltene Alkoxylierungsgrad beispielsweise im Bereich von 2 bis 20, bevorzugt im Bereich von 2,5 bis 14, besonders bevorzugt im Bereich von 3 bis 6 liegt.

### BEISPIELE

### Herstellbeispiel: Katalysator

In einem Rührkessel mit einem Volumen von 30 1, ausgestattet mit einem Propellerrührer, Tauchrohr für die Dosierung, pH-Sonde und Streulicht-Sonde, wurden 16000 g wässrige Hexacyanocobaltsäure (Cobalt-Gehalt: 9 g/l) vorgelegt und unter Rühren auf 50°C erwärmt. Anschließend wurden unter Rühren mit einer Rührleistung von 0,4 W/l 9224 g wässrige Zinkacetat-Dihydrat-Lösung (Zink-Gehalt: 2,6 Gew.-%), welche auf ebenfalls 50°C temperiert war, innerhalb von 15 Minuten zugefahren.

Zu dieser Fällsuspension wurden 351 g Pluronic® PE 6200 (BASF AG) zugesetzt und die Mischung weitere 10 Minuten gerührt.

Anschließend wurden weitere 3690 g wässrige Zinkacetat-Dihydrat-Lösung (Zink-Gehalt: 2,6 Gew.-%) unter Rühren mit einer Rührenergie von 1 W/l innerhalb 5 Minuten zudosiert.

Die Suspension wurde zwei Stunden nachgerührt. Der pH-Wert fiel in dieser Zeit von 4,02 auf 3,27 und blieb dann konstant. Die so erhaltene Fällsuspension wurde anschließend abfiltriert und auf dem Filter mit dem 6-fachen Kuchenvolumen an Wasser gewaschen.

Der feuchte Filterkuchen wurde getrocknet und mittels Spalt-Rotor-Mühle in Tridekanol® N dispergiert. Die dabei erhaltene Suspension hatte einen Multimetallcyanidgehalt von 5 Gew.-%.

### Vergleichsbeispiel 1: Anspringverhalten

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator wurden bei einer Temperatur von 100 °C und ca. 20 mbar für zwei Stunden in einem 2-1-Druckautoklaven entwässert. Anschließend wurde dreimal mit Stickstoff gespült und danach auf die gewünschte Temperatur erhitzt. Nach Erreichen der Temperatur wurden unter Rühren insgesamt 30 g Ethylenoxid zudosiert. Es wurde die Zeit (Induktionszeit) bestimmt vom Beginn der Dosierung bis zum Beginn der Reaktion. Der Start der Reaktion ist an der damit verbundenen Wärmeentwicklung und somit Erhöhung der Temperatur der Reaktionsmischung erkennbar.

| *Temperatur* | *Induktionszeit* |
|---|---|
| 100 °C | nicht angesprungen |
| 120 °C | 20 min |
| 140 °C | 5 min |
| 160°C | 5 min |

### Vergleichsbeispiel 2 (2-Propylheptanol + 8 EO bei 160 °C)

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 160 °C erhitzt. Nach Erreichen der Temperatur sollten unter Rühren insgesamt 704 g (16,0 Mol) Ethylenoxid zudosiert werden. Nach Zugabe von 572 g Ethylenoxid war keine ausreichende Reaktion mehr feststellbar (kaum Druckabnahme, kaum Wärementwicklung).

### Vergleichsbeispiel 3 2-Propylheptanol + 1,2 PO + 6 EO bei 160 °C)

316 g (2,0 Mol) 2-Propyl-Heptanol-1 und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 160 °C erhitzt. Nach Erreichen der Temperatur wurden unter Rühren insgesamt 140 g (2,4 Mol) Propylenoxid bei 160 °C zudosiert. Nach Ende der PO-Dosierung rührte man noch 15 Minuten bei 160 °C. Danach sollten 528 g (12,0 Mol) Ethylenoxid zudosiert werden. Nach Zugabe von 444 g Ethylenoxid war keine ausreichende Reaktion mehr feststellbar (kaum Druckabnahme, kaum Wärementwicklung).

### Beispiel 1(2-Propylheptanol + 8 EO bei 140 °C)

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 704 g (16,0 Mol) Ethylenoxid zudosiert. Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 2 (reines 2-Propylheptanol + 1,2 PO + 6 EO bei 140 °C)

316 g (2,0 Mol) 2-Propyl-Heptanol-1 und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 140 g (2,4 Mol) Propylenoxid bei 140 °C zudosiert. Nach Ende der PO-Dosierung rührte man noch 15 Minuten bei 140 °C und begann dann mit der Dosierung von insgesamt 528 g (12,0 Mol) Ethylenoxid. Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 3(2-Propylheptanol + 1,2 PO + 6 EO bei 140 °C und max. 2,0 bar EO-Druck)

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 140 g (2,4 Mol) Propylenoxid bei 140 °C zudosiert. Nach Ende der PO-Dosierung rührte man noch 15 Minuten bei 140 °C.

Dann stellte man einen Gesamtdruck von 2,0 bar Stickstoff (absolut) bei 140 °C ein und begann danach mit der Dosierung von insgesamt 528 g (12,0 Mol) Ethylenoxid bei einem Gesamtdruck von maximal 4,0 bar (absolut, 140 °C). Nach beendeter Ethylenoxid-Dosierung rührte man noch 1 h bei 140 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 4(2-Propylheptanol + 8 EO bei 150°C und 2,25 bar EO-Druck)

316 g (2,0 Mol) 2-Propyl-Heptanol-1 und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach ein Gesamtdruck von 2,25 bar Stickstoff (absolut) bei 150 °C eingestellt. Nach Erreichen der Temperatur wurden unter Rühren insgesamt 704 g (16,0 Mol) Ethylenoxid bei einem Gesamtdruck von maximal 4,5 bar (absolut bei 150 °C) zudosiert. Nach beendeter Ethylenoxid-Dosierung rührte man noch 1 h bei 150 °C, kühlte danach auf 80 °C ab, spülte dreimal mit Stickstoff, evakuierte dann zum Entgasen auf 20 mbar, und entleerte den Reaktor. Das Reaktionsprodukt wurde nicht filtriert und entsprach dem gewünschten Produkt.

### Beispiel 5 (2-Propylheptanol + 0,8 PO) (nicht erfindungsgemäß)

316 g (2,0 Mol) 2-Propyl-Heptanol-1 (Isomerengemisch aus 87% 2-Propy)heptano)-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 93 g (1,6 Mol) Propylenoxid bei 140 °C zudosiert. Nach Ende der PO-Dosierung rührte man noch 15 Minuten bei 140 °C, spülte dreimal mit Stickstoff, evakuierte dann zum Entgasen auf 20 mbar, kühlte danach auf 80 °C ab, und entleerte den Reaktor.

| | |
|---|---|
| Restalkoholgehalt (2-Propyl-heptanol-1): | 28,6% |

### Beispiel 6 (2-Propylheptanol + 1.0 PO) (nicht erfindungsgemäß)

Es wurde vorgegangen wie in Beispiel 5. Allerdings gab man 2,0 Mol Propylenoxid statt 1,6 Mol zu und arbeitete bei einer Reaktionstemperatur von 140 °C.

| | |
|---|---|
| Restalkoholgehalt (2-Propyl-heptanol-1): | 24,2% |

### Beispiel 7 (2-Propylheptanol + 1,20 PO) (nicht erfindungsgemäß)

Es wurde vorgegangen wie in Beispiel 5. Allerdings gab man 2,4 Mol Propylenoxid statt 1,6 Mol zu und arbeitete bei einer Reaktionstemperatur von 160°C.

| | |
|---|---|
| Restalkoholgehalt (2-Propyl-heptanol-1): | 20,0% |

### Beispiel 8 (2-Propylheptanol + 1,20 PO) (nicht erfindungsgemäß)

Es wurde vorgegangen wie in Beispiel 5. Allerdings gab man 2,4 Mol Propylenoxid statt 1,6 Mol zu und arbeitete bei einer Reaktionstemperatur von 140 °C.

| | |
|---|---|
| Restalkoholgehalt (2-Propyl-heptanol-1): | 19,8% |

### Beispiel 9 (2-Propylheptanol + 1,23 PO) (nicht erfindungsgemäß)

Es wurde vorgegangen wie in Beispiel 5. Allerdings gab man 2,46 Mol Propylenoxid statt 1,6 Mol zu und arbeitete bei einer Reaktionstemperatur von 120 °C.

| | |
|---|---|
| Restalkoholgehalt (2-Propyl-heptanol-1): | 20,8% |

### Beispiel 10 (2-Propylheptanol + 1,28 PO) (nicht erfindungsgemäß)

Es wurde vorgegangen wie in Beispiel 5. Allerdings gab man 2,56 Mol Propylenoxid statt 1,6 Mol zu und arbeitete bei einer Reaktionstemperatur von 140 °C.

| | |
|---|---|
| Restalkoholgehalt (2-Propyl-heptanol-1): | 17,7% |

### Beispiel 11 (2-Propylheptanol + 1,30 PO) (nicht erfindungsgemäß)

Es wurde vorgegangen wie in Beispiel 5. Allerdings gab man 2,6 Mol Propylenoxid statt 1,6 Mol zu und arbeitete bei einer Reaktionstemperatur von 140 °C.

| | |
|---|---|
| Restalkoholgehalt (2-Propyl-heptanol-1): | 17,6% |

### Beispiel 12 (2-Propylheptanol + 1,40 PO) (nicht erfindungsgemäß)

Es wurde vorgegangen wie in Beispiel 5. Allerdings gab man 2,8 Mol Propylenoxid statt 1,6 Mol zu und arbeitete bei einer Reaktionstemperatur von 140 °C.

| | |
|---|---|
| Restalkoholgehalt (2-Propyl-heptanol-1): | 15,8% |

### Beispiel 13 (2-Propylheptanol + 1,44 PO) (nicht erfindungsgemäß)

Es wurde vorgegangen wie in Beispiel 5. Allerdings gab man 2,88 Mol Propylenoxid statt 1,6 Mol zu und arbeitete bei einer Reaktionstemperatur von 140 °C.

| | |
|---|---|
| Restalkoholgehalt (2-Propyl-heptanol-1): | 14,8% |

### Beispiel 14 (2-Propylheptanol + 1,51 PO) (nicht erfindungsgemäß)

Es wurde vorgegangen wie in Beispiel 5. Allerdings gab man 3,02 Mol Propylenoxid statt 1,6 Mol zu und arbeitete bei einer Reaktionstemperatur von 120 °C.

| | |
|---|---|
| Restalkoholgehalt (2-Propyl-heptanol-1): | 15,0% |

### Beispiel 15 (2-Propylheptanol + 1,63 PO) (nicht erfindungsgemäß)

Es wurde vorgegangen wie in Beispiel 5. Allerdings gab man 3,26 Mol Propylenoxid statt 1,6 Mol zu und arbeitete bei einer Reaktionstemperatur von 160 °C.

| | |
|---|---|
| Restalkoholgehalt (2-Propyl-heptanol-1): | 10,1% |

### Beispiel 16 (2-Propylheptanol + 1,71 PO) (nicht erfindungsgemäß)

Es wurde vorgegangen wie in Beispiel 5. Allerdings gab man 3,42 Mol Propylenoxid statt 1,6 Mol zu und arbeitete bei einer Reaktionstemperatur von 120 °C.

| | |
|---|---|
| Restalkoholgehalt (2-Propyl-heptanol-1) | 10,7% |

## Patentansprüche

1. Verfahren zur Herstellung mindestens eines Alkoxylats, umfassend das Inkontaktbringer von Ethylenoxid, mit 2-Propylheptanol oder einem Isomerengemisch davon, in Gegenwa mindestens einer Doppelmetallcyanid-Verbindung der allgemeinen Formel I:
M¹ₐ[M²(CN)_{b}(A)_{c}]_{d}·fM¹_{g}Xₙ·h(H₂O)·eL·kP (I),
in der
- M¹ mindestens ein Metallion, ausgewählt aus der Gruppe bestehend aus Zn²⁺, Fe²⁺, Fe³⁺, Co³⁺, Nl²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Tl³⁺, Tl⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Rh³⁺, Ru²⁺, Ir³+ist,
- A und X unabhängig voneinander ein Anion, ausgewählt aus der Gruppe bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochene oder ganze Zahl größer 0 oder 0 ist,
- f und h unabhängig voneinander eine gebrochene oder ganze Zahl größer 0 oder 0 sind
**dadurch gekennzeichnet, dass** die Umsetzung bei einer Temperatur von 130°C bis 155°C erfolgt, und ein Inertgas zugegeben wird, wobei der Partialdruck des Inertgases während der Umsetzung 1,5 bis 20 bar beträgt.

2. Verfahren nach Anspruch 1, **dadurch gekennzeichnet, dass** mindestens eine der folgenden Eigenschaften erfüllt ist:
(1) M¹ ist ausgewählt aus der Gruppe Zn²⁺, Fe²⁺, Fe³⁺, Co³⁺, Nl²⁺, Mn²⁺, Co²⁺;
(2) M² ist ausgewählt aus der Gruppe Fe²⁺, Fe³⁺, Co³⁺.

3. Verfahren nach einem der Ansprüche 1 oder 2, **dadurch gekennzeichnet, dass** M¹ Zn²⁺ und M² Co³⁺ ist.

4. Verfahren nach einem der Ansprüche 1 bis 3, **dadurch gekennzeichnet, dass** während der Induktionsphase die Summe aus Inertgas-Partialdruck und Alkylenoxid-Partialdruck bei 1,5 bar bis 6,0 bar liegt.

## Claims

1. A process for preparing at least one alkoxylate, which comprises bringing ethylene oxide into contact with 2-propylheptanol or an isomer mixture thereof in the presence of at least one double metal cyanide compound of the general formula I:
M¹ₐ[M²(CN)_{b}(A)_{c}]_{d}·fM¹_{g}Xₙ·h(H₂O)·eL·kP (I),
where
- M¹ is at least one metal ion selected from the group consisting of Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
- M² is at least one metal ion selected from the group consisting of Fe²⁺, Fe³⁺, Co²⁺, Co³⁺, Mn²⁺, Mn³⁺, V⁴⁺, V⁵⁺, Cr²⁺, Rh³⁺, Ru²⁺, Ir³⁺,
- A and X are each, independently of one another, an anion selected from the group consisting of halide, hydroxide, sulfate, carbonate, cyanide, thiocyanate, isocyanate, cyanate, carboxylate, oxalate, nitrate, nitrosyl, hydrogensulfate, phosphate, dihydrogenphosphate, hydrogenphosphate and hydrogencarbonate,
- L is a water-miscible ligand selected from the group consisting of alcohols, aldehydes, ketones, ethers, polyethers, esters, polyesters, polycarbonate, ureas, amides, primary, secondary and tertiary amines, ligands having a pyridine nitrogen, nitriles, sulphides, phosphides, phosphites, phosphanes, phosphonates and phosphates,
- k is a fraction or integer greater than or equal to zero, and
- P is an organic additive,
- a, b, c, d, g and n are selected so that the compound (I) is electrically neutral, with c being able to be 0,
- e is the number of ligand molecules and is a fraction or integer greater than 0 or is 0,
- f and h are each, independently of one another, a fraction or integer greater than 0 or 0,
wherein the reaction is carried out at a temperature of from 130°C to 155°C, and an inert gas is added, wherein the inert gas partial pressure during the reaction is 1.5 to 20 bar.

2. The process according to claim 1, wherein at least one of the following properties is fulfilled:
(1) M¹ is selected from the group consisting of Zn²+, Fe²⁺, Fe³⁺, Co³+, Ni²+, Mn²+, Co²⁺;
(2) M² is selected from the group consisting of Fe²⁺, Fe³⁺, Co³⁺.

3. The process according to claim 1 or 2, wherein M¹ is Zn²⁺ and M² is Co³⁺.

4. The process according to any of claims 1 to 3, wherein the sum of inert gas partial pressure and alkylene oxide partial pressure is from 1.5 bar to 6.0 bar during the induction phase.

## Revendications

1. Procédé de préparation d'au moins un alcoxylate, comprenant la mise en contact de l'oxyde d'éthyène, avec 2-propylheptanol ou un mélange d'isomères de celui-ci, en présence d'au moins un composé de cyanure bimétallique de la formule générale I:
M¹ₐ[M²(CN)_{b}(A)_{c}]_{d}·fM¹_{g}Xₙ·h(H₂O)·eL·kP (I),
dans laquelle:
- M¹ est au moins un ion métallique, choisi dans le groupe formé par Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺,
- M² est au moins un ion métallique, choisi dans le groupe formé par Fe²⁺, Fe³⁺, Co²⁺, Co³⁺, Mn²⁺, Mn³⁺, V⁴⁺, V⁵⁺, Cr²⁺, Rh³⁺, Ru²⁺, Ir³⁺,
- A et X représentent indépendamment l'un de l'autre un anion, choisi dans le groupe formé par des anions halogénure, hydroxide, sulfate, carbonate, cyanure, thiocyanate, isocyanate, cyanate, carboxylate, oxalate, nitrate, nitrosyle, hydrogénosulfate, phosphate, dihydrogénophosphate, hydrogénophosphate ou hydrogénocarbonate,
- L est un ligand miscible à l'eau, choisi dans le groupe formé par des alcools, des aldéhydes, des cétones, des éthers, des polyéthers, des esters, des polyesters, du polycarbonate, des urées, des amides, des amines primaires, secondaires et tertiaires, des ligands avec de l'azote de pyridine, des nitriles, des sulfures, des phosphures, des phosphites, des phosphanes, des phosphonates et des phosphates,
- k est un nombre fractionnaire ou entier supérieur ou égal à zéro, et
- P est un additif organique,
- a, b, c, d, g et n sont choisis de manière que l'électroneutralité du composé (I) soit garantie, c pouvant être égal à 0,
- e est le nombre des molécules de ligand, un nombre entier ou fractionnaire supérieur à 0 ou égal à 0,
- f et h représentent indépendamment l'un de l'autre un nombre fractionnaire ou entier supérieur à 0 ou ègal à 0,
**caractérisé en ce que** la réaction a lieu à une température de 130°C à 155°C, et on ajoute un gaz inerte avec une pression partielle du gaz inerte pendant la réaction de 1,5 à 20 bar.

2. Procédé suivant la revendication 1, **caractérisé en ce qu'**au moins l'une des propriétés suivantes est remplie:
(1) M¹ est choisi dans le groupe formé par Zn²⁺, Fe²⁺, Fe³+, Co³⁺, Ni²⁺, Mn²⁺, Co²⁺;
(2) M² est choisi dans le groupe formé par Fe²⁺, Fe³⁺, Co³⁺.

3. Procédé suivant l'une quelconque des revendications 1 ou 2, **caractérisé en ce que** M¹ est du Zn²⁺ et M² est du Co³⁺.

4. Procédé suivant l'une quelconque des revendications 1 à 3, **caractérisé en ce que**, pendant la phase d'induction, la somme de la pression partielle de gaz inerte et de la pression partielle de l'oxyde d'alkylène est de 1,5 bar à 6,0 bars.
